# EUROPEAN PATENT SPECIFICATION

(11) **EP 1 513 541 B1**
(45) Date of publication and mention of the grant of the patent: **28.01.2009**
(21) Application number: 03760972.4
(22) Date of filing: 20.06.2003
(51) Int. Cl.: A61K 35/74, A23L 1/03, A23L 1/308

(54) **METHOD FOR PREVENTING OR ALLEVIATING SYMPTOMS OF MALABSORPTION FROM THE GASTROINTESTINAL TRACT**
METHODE ZUR VERHINDERUNG ODER LINDERUNG VON SYMPTOMEN BEI UNZUREICHENDER AUFNAHME AUS DEM GASTROINTESTINALTRAKT
PROCEDE DESTINE A EMPECHER OU A REMEDIER AUX SYMPTOMES DE MALABSORPTION

(30) Priority: 20.06.2002 EP 02077460
(43) Date of publication of application: 16.03.2005
(60) Divisional application: 08157093.9
(73) Proprietor: N.V. Nutricia, 2700 MA Zoetermeer (NL)
(72) Inventor: SPEELMANS, Gelske, NL-6721 SM Bennekom (NL); KNOL, Jan, NL-6708 SM Wageningen (NL); GOVERS, Maria, Johanna, Adriana, Petronella, NL-6706 EV Wageningen (NL)
(74) Representative: van Westenbrugge, Andries
(86) International application number: PCT/NL2003/000455
(87) International publication number: WO 2004/000340

(56) References cited:
- EP-A- 0 914 778
- EP-A- 1 216 706
- WO-A-00/20013
- WO-A-01/05413
- WO-A-02/060276
- FR-A- 2 741 509
- US-A- 5 478 557
- US-A- 6 077 504
- VORAGEN AGJ: "Cutting-edge carbohydrates" PREPARED FOODS, vol. 169, no. 5, May 2000 (2000-05), pages 137-138, XP009002318
- SARKAR S ET AL: "PROCESS FOR THE MANUFACTURE OF A NEW MODIFIED CULTURED MILK PRODUCT FOR INFANTS AND CHILDREN" MILCHWISSENSCHAFT, VV GMBH VOLKSWIRTSCHAFTLICHER VERLAG. MUNCHEN, DE, vol. 53, no. 11, 1998, pages 603-605, XP000799389 ISSN: 0026-3788
- RINTA-KOSKI M; BEASLEY S; MONTONEN L; MANTERE-ALHONEN S: "Propionibacteria isolated from fumen used as possible probiotics together with bifidobacteria" MILCHWISSENSCHAFT, vol. 56, no. 1, January 2001 (2001-01), pages 11-13, XP009002154
- MANTERE-ALHONEN: "Propionicbacteria used as probiotics - a review" LAIT, vol. 75, no. 4-5, 1995, pages 447-452, XP009002175
- YOUNG J: "European market developments in prebiotic- and probiotic- containing foodstuffs" THE BRITISH JOURNAL OF NUTRITION, vol. 80, no. 4, October 1998 (1998-10), pages S231-S233, XP009002339
- MORI H ET AL: "ISOLATION AND STRUCTURAL IDENTIFICATION OF BIFIDOGENIC GROWTH STIMULATOR PRODUCED BY PROPIONIBACTERIUM FREUDENREICHII" JOURNAL OF DAIRY SCIENCE, XX, US, vol. 80, no. 9, 1997, pages 1959-1964, XP009002165 ISSN: 0022-0302

## Description

### TECHNICAL FIELD

The present invention relates to a method of preventing or alleviating symptoms of malabsorption. The present method comprises enteral administration to a monogastric mammal of a composition that enhances digestion of nutrients in the gastrointestinal tract and is particularly suitable for treating patients suffering from cystic fibrosis, pancreatic insufficiency, coeliac disease, cow's milk protein allergy, small or short bowel syndrome, or small bowel overgrowth.

### BACKGROUND OF THE INVENTION

A principal function of the gastrointestinal tract is to process and absorb food. The stomach, which is both a storage and digestive organ, works to optimise the conditions for the digestion and absorption of food in the small intestine. Following the stomach and preceding the large bowel (colon) is the small intestine which comprises three regions: the duodenum, jejunum, and ileum. A major function of the small intestine is one of absorption of digested nutrients.

The passage of food through the gastrointestinal tract, which leads to digestion and absorption of the nutrients contained therein, is controlled by a complex system of inhibitory and stimulatory motility mechanisms which are set in motion by the composition of the meal ingested. Specific receptors for fats, and proteins, and the osmolality, acidity and particle size of the meal activate propulsive and inhibitory reactions, which modulate transit and thus absorption.

Disruption of the normal digestive and absorptive processes frequently manifests as a variety of syndromes, such as, for example malabsorption, weight loss, diarrhoea, vitamin deficiency, electrolyte imbalance, and the like. The malabsorption syndromes are clinical entities associated with defective absorption of amino acids, sugar, fat, vitamins and minerals. Malabsorption is thus the common denominator in a wide variety of clinical disorders the severity of which covers a broad range and which, when diagnosed, require individual analysis of each patient against the background of the normal process of absorption. Patients with malabsorption syndrome have a difficult time meeting daily nutritional requirements. In patients with malabsorption syndrome there generally exists both catabolic and serious nutritional consequences.

The efficiency with which nutrients are absorbed (and thus utilised) by the human and animal body depends among other things on the efficiency of digestion. Digestion is *inter alia* mediated by various enzymes that have specific functions at various locations in the digestive tract. Impairment of digestion, e.g. as a result of diminished intestinal enzyme activity, is one of the prime causes of malabsorption.

In the whole process of digestion, which starts in the mouth, the pancreas has an important role. It secretes a juice having two major components, an alkaline fluid and enzymes, into the duodenum. The two components occur in variable proportions depending on the stimuli. The alkaline fluid component, ranging in volume from 200-800 ml/day, has a high concentration of bicarbonate, which neutralises the gastric content entering the duodenum and helps to regulate the pH of the intestinal tract.

The enzymes of pancreatic juice are synthesised and secreted by the acinar cells which also secrete a fluid similar in electrolyte composition to an ultrafiltrate of plasma. In intermittent feeders such as man, dog and cat, the volume of the fluid secreted by the acinar cells is very small and it has little effect upon the volume and composition of pancreatic juice flowing in the main pancreatic duct.

The pancreatic juice contains four major enzyme groups: lipolytic, proteolytic, amylolytic and nucleic acid splitting enzymes. These pancreatic enzymes, some of them are secreted in multiple forms, possess specifities complementary to the intestinal membrane bound enzymes. Fresh, uncontaminated pancreatic juice is without proteolytic activity because these enzymes are in the form of inactive zymogens. An important fraction of the calcium in pancreatic juice accompanies the enzymes, especially alpha-amylase.

Patients suffering from malabsorption as a result of diminished intestinal enzyme activity (e.g. as a result of pancreatic insufficiency), are commonly treated by orally providing one or more digestive enzymes (exocrine pancreas substitution therapy). An important aim of exocrine pancreas substitution therapy is to eliminate the malabsorption and to maintain an adequate nutrition. However it is known that in many patients suffering from pancreatic insufficiency, exocrine pancreas substitution therapy will not restore digestive efficacy to normal levels. As a result, many patients using exocrine pancreas substitution therapy still suffer from symptoms of malabsorption.

Malabsorption as a result of impaired digestion may also occur in subjects who do not suffer from pancreatic insufficiency, e.g. in patients suffering from small or short bowel syndrome. In patients suffering from short bowel syndrome the passage of nutrients through the small intestine occurs in such a short time interval that nutrients are only partly digested before they enter the colon. Exocrine pancreas substitution therapy is not effective in such patients and alternative methods of treatment are generally inadequate in enhancing the digestive processes in these patients.

Thus, despite the tremendous amount of research that has been undertaken to elucidate the role of nutrition and absorption in gastrointestinal disorders, few methods currently exist that offer adequate solace for patients suffering from these disorders, in particular patients suffering from malabsorption as a result of impaired intestinal digestion. Thus, a need exists for new methods and nutritional compositions that can effectively be employed to improve digestion and subsequent absorption of ingested nutrients.

### SUMMARY OF THE INVENTION

The inventors have found that symptoms of malabsorption in monogastric mammals which is caused by impaired intestinal digestion may effectively be alleviated or prevented by enterally administering an effective amount of a prebiotic that stimulates gastrointestinal activity of *Propionibacterium.*

*Propionibacterium* is found in rumen and intestine of ruminants (cattle, sheep), where it plays a part in the formation of fatty acids, particularly propionic and acetic acids. *Propionibacterium* is responsible for the conversion of lactate, which arises from various fermentations in the rumen, to predominantly propionate. Under anaerobic conditions, members of the genus *Propionibacterium* ferment glucose, sucrose, lactose and pentoses, as well as lactate, malate, glycerol and other substrates to propionic acid. The catabolism of hexoses proceeds via the fructose-bisphosphate pathway.

Although the inventors do not wish to be bound by theory, it is believed that in the anaerobic intestinal environment *Propionibacterium* is capable of fermenting nutrients, which the mammal is unable to (enzymatically) digest and/or absorb, to propionic acid (or propionate). Propionic acid is readily absorbed in the intestine, following which it can be used as an energy source. Thus, the enteral administration of *Propionibacterium* enables the mammal to recover a larger fraction of its total energy intake in the form of ingested food Similarly, because *Propionibacterium* naturally occur in the intestinal microflora, the enteral administration of a prebiotic that enhances the intestinal activity *of Propionibacterium* may be used to achieve the same effect. Since the digestion and absorption of various nutrients is strongly interrelated, the enteral administration of *Propionibacterium* and/or the prebiotic is also believed to improve the mammal's capability of satisfying its needs for certain essential nutrients.

Other micro-organisms, such as those belonging to the genus *Lactobacillus* and *Enterococcus,* are known to also have the capability to ferment nutrients to short chain fatty acids within the intestinal tract. However, the inventors have found that *Propionibacterium* offers several important advantages over these other micro-organisms.

It was found that, unlike micro-organisms such as *Lactobacillus* and *Enterococcus and Bifidobacterium* which are also capable of fermenting nutrients to short chain fatty acids, *Propionibacterium* ferments nutrients to propionic acid without simultaneously producing substantial amounts of methane. Whereas *Propionibacterium* only produce propionic acid, all other foodgrade micro-organisms produce lactate and/or acetate which are easily converted to methane. This means that, in comparison to these other micro-organisms, *Propionibacterium* will give rise to significantly less flatulence and bloating. In addition, it is important to note that fermentative production of methane is associated with less energy yield for the mammal, meaning that *Propionibacterium* is capable of providing more energy sources for the mammal from the same substrate than these other micro-organisms.

Thus, the present method of enteral administration to monogastric mammals of a prebiotic that stimulates gastrointestinal activity of *Propionibacterium* is a very effective method for treating or preventing symptoms of malabsorption caused by impaired intestinal digestion as the method is particularly effective in enhancing energy recovery from ingested nutrients and does not suffer from unpleasant side-effects such as flatulence and bloating.

The present method is of general benefit to monogastric mammals who are malnutritioned, or at risk of becoming malnutritioned as a result of malabsorption. It is of crucial importance for such mammals to recover as much energy as possible from ingested nutrients. The present invention enables enhanced energy recovery from ingested nutrients, which is advantageous to any mammal suffering from malabsorption as a result of impaired enzymatic or fermentative intestinal digestion, even if such malabsorption is not caused by maldigestion.

### DETAILED DESCRIPTION OF THE INVENTION

Consequently, one aspect of the invention relates to a method of preventing or alleviating symptoms of malabsorption in a monogastric mammal which malabsorption is caused by impaired fermentative intestinal digestion, said method comprising the enteral administration to said mammal of an effective amount of a prebiotic that stimulates gastrointestinal activity of *Propionibacterium,* and optionally one or more strains of the genus *Propionibacterium*.

The term "monogastric mammal" encompasses all mammals that do not belong to the group of polygastric mammals. Important representatives of the group of polygastric mammals are ruminants such as cattle. Typical examples of monogastric mammal include humans, pigs, dogs and cats.

The term "enteral administration" includes oral and rectal administration The term "oral administration" also encompasses tube feeding. Preferably, the present method employs oral administration.

The term "malabsorption" refers to a condition of defective intestinal absorption of amino acids, peptides, sugar, fat, vitamins and/or minerals. Preferably, malabsorption is defined more narrowly as defective intestinal absorption of amino acids peptides, degradation products of carbohydrates and/or degradation products of fatt.

The effectiveness of the present method is dependent on the amount of *Propionibacterium* cells and/or prebiotic administered as well as on the status of the *Propionibacterium* cells. In a particularly preferred embodiment metabolically active cells of *Propionibacterium* are administered in a daily amount of at least 10⁶ cells. More preferably metabolically active cells are administered in a daily amount of at least 10⁸ cells, most preferably of at least 10⁹ cells. Here the term "daily amount" refers to the average daily amount. The term "metabolically active" refers to the capability of the cells to convert ingested nutrients to propionic acid.

In general, the prebiotic will be administered in a daily amount of at least 0.5 g, preferably in a daily amount of at least 1 g, most preferably of at least 5 g. The daily administered amount of prebiotic will usually not exceed 50 g. Preferably said amount will not exceed 30 g.

In a very preferred embodiment of the invention, the present method comprises the co-administration of *Propionibacterium* and the prebiotic. This particular embodiment not only offers the advantage that it introduces one or more metabolically active strains of *Propionibacterium* into the intestine but, in addition, it stimulates metabolic activity and even proliferation of these strains at the cost of other, less desirable, micro-organisms present in the intestinal microflora.

The inventors have discovered that the impact of the present method on the ratio of propionate to acetate found in the faeces of the mammal is a reliable indicator of the effectiveness of the present method. High priopionate to acetate ratios generally correspond with high energy recovery and little methane production as such a high ratio is indicative of high intestinal activity of *Propionibacterium,* relative to the intestinal activity of other micro-organisms. The ratio in which propionate and acetate occur in faeces may be influenced effectively by the administration of a suitable prebiotic, i.e. a substrate for *Propionibacterium.* The terms "propionate" and "acetate" as used in this document encompass both the free acid and the salt forms of these substances.

In order to establish whether or not a component can suitably be used as a prebiotic in the present method a relatively simple *in vitro* test can be applied. This method is described in detail in appendix I.

In a particularly preferred embodiment, the prebiotic used in accordance with the invention yields a propionate to acetate ratio which is at least 25% higher than that observed for a glucose control in the aforementioned *in vitro* incubation test. More preferably said ratio is at least 50% higher and most preferably it is at least 75% higher than the same ratio observed for the glucose controL

In the present method, the frequency of administration may suitably vary from 4 times daily to once a week. Preferably the frequency is between 3 times a day and once every 3 days. Most preferably the frequency of administration is once or twice daily. The present method is particularly effective when it comprises uninterrupted administration of *Propionibacterium* and/or the prebiotic during a period of at least 1 week, more preferably of at least 3 weeks.

The prebiotics that may advantageously be employed in the present method, because they are good sources of propionate, are inulin; modified inulin; hydrolysed inulin; fructo-oligosaccharides; galactomannan (e.g. guar gum) and hydrolysates thereof; arabinogalactan (e.g. gum acacia) and hydrolysates therof; trans-galacto-oligosaccharides; rhamnose; pectin and pectin hydrolysates; resistant starch and hydrolysates thereof; indigestible dextrin and hydrolysates thereof; indigestible polydextrose; beta-glucan and hydrolysates thereof; and combinations of these oligo- and polysaccharides. Preferably, the prebiotic is selected from the group consisting of inulin; modified inulin; hydrolysed inulin; fructo-oligosaccharides ; arabinogalactan and hydrolysates therof; resistant starch; indigestible dextrin; indigestible polydextrose; and combinations thereof. More preferably, the prebiotic is selected from the group consisting of inulin; modified inulin; hydrolyses inulin; fructo-oligosaccharides; arabinogalactan and hydrolysates thereof; resistant starch; indigestible dextrin and combinations thereof Most preferably, the prebiotic is selected from the group consisting of inulin, arabinogalactan, resistant starch, indigestible dextrin and combinations thereof.

Screening studies conducted by applicant using the method described in Appendix 1 showed that the following carbohydrates are particularly suitable prebiotics:

| **Prebiotic** | **Propionate/acetate ratio relative to glucose control (after 48 hours)** |
|---|---|
| Resistant starch (Novelose™ 330) | 4.77 |
| Indigestible dextrin (Fibersol™) | 3.46 |
| Arabinogalactan (acacia) | 2.02 |
| Inulin (Raftline™) | 2.02 |

For nutritional purposes, starch can be classified into three categories: rapidly digestible, slowly digestible and resistant starch. Resistant starches pass the small intestines without being digested or absorbed. Subsequently, in the large intestine, the resistant starch can be broken down by fermentation to short chain fatty acids, carbon dioxide, hydrogen and methane. There are four types of resistant starches:
1. physically inaccessible starches that are locked away within cell walls such as those found in partially milled grains, seeds and legumes
2. native resistant starch granules like those typically found in bananas, raw potatoes and high amylase maize starch
3. retrograded crystalline non-granular starch, like starch found in cooked and cooled, potatoes, bread crusts, cornflakes and retrograded high amylase maize starch
4. specific chemically modified or repolymerised starch such as chain linked altered dextrins (see article by M. Croghan in Functional Foods & Nutraceuticals; September/October 2001)

In another preferred embodiment of the invention *Propionibacterium* is administered in the form of viable cells, i.e. cells that are capable of replication. The present method advantageously comprises the enteral administration of one or more viable strains of the genus *Propionibacterium* in a daily amount of at least 10⁶ CFU, preferably of at least 10⁸ CFU, most preferably of at least 10⁹ CFU.

Examples of species *of Propionibacterium* that may suitably be employed in the present process include *P. freudenreichii, spp freudenreichii* or *spp shermanii; P. jenseiiii; P. acidipropionici* and *P. microaerophilus.*

The present method advantageously comprises the enteral co-administration of one or more metabolically active homofermentative strains of the genus *Lactococcus* or *Lactobacillus,* preferably in a daily amount of at least 10⁶ cells. Homofermentative strains of the genus *Lactococcus or Lactobacillus* are capable of anaerobic fermentation of nutrients to lactic acid and, unlike heterofermentative strains, produce virtually no carbon dioxide. The co-administration of the genus *Lactococcus* and/or *Lactobacillus* offers the advantage that even higher energy recovery rates can be achieved as the latter strains are particularly efficient in fermenting certain nutrients such as lactose. The fermentation products of *Lactococcus* and/or *Lactobacilitis,* especially lactate, may suitably serve as a substrate for the *Propionibacterium* strain(s).

The strains of the genus *Lactobacillus* are preferably selected from the group consisting of *L. casei, L. plantarum, L. sake, L. salivarius, L. acidophilus, L. crispatus, L. johnsonii, L. gasseri, L. delbrueckii, L. jensenii, L. curvatus, L. helveticus, L. amilophilus, L. amylovorus* and *L. rhamnosus.*

Another genus of micro-organisms that may advantageously be co-administered with *Propionibacterium* is *Bifidobacterium. Bifidobacterium* is highly efficient in degrading (hydrolysing) complex carbohydrates which may subsequently be fermented by *Propionibacterium* and/or *Lactococcus*/*Lactobacillus.* Consequently, in a preferred embodiment, the present method comprises the enteral co-administration of one or more metabolically active strains of the genus *Bifidobacterium,* preferably in a daily amount of at least 10⁶ cells, more preferably in a daily amount of at least 10⁸ cells.

The present method may suitably be used to treat patients suffering from various forms of malabsorption. The method is Particularly effective for treating patients who suffer from malabsorption caused by impaired enzymatic or fermentative intestinal digestion. Best results are obtained in patients suffering from some form of pancreatic insufficiency or short bowel syndrome.

The enteral administration *of Propionibacterium* can provide a remedy for defective intestinal absorption of amino acids, peptides, sugars, fat, vitamins and/or minerals. The present method is particularly effective for treating mammals suffering from malabsorption of lipids and/or carbohydrates. A crucial benefit of the present method is that it enhances the energy that the treated mammal can derive from ingested nutrients, especially ingested carbohydrate material.

The symptoms of malabsorption treated by the present method may be associated with a variety of disorders. The present method is very suitable for treating patients who suffer from malabsorption which is associated with a disorder selected from the group consisting of cystic fibrosis, pancreatic insufficiency, pancreatitis, diseases accompanied by villous atrophy (e.g. caused by coeliac disease, cow's milk protein allergy, α chain disease, and inflammatory or infectious diseases), small or short bowel syndrome, malabsorption of mono- and disaccharides, and small bowel (bacterial) overgrowth. The method of the invention is particularly suitable for treating or preventing symptoms of malabsorption in patients suffering from a disorder selected from the group consisting of cystic fibrosis, pancreatic insufficiency, pancreatitis or small or short bowel syndrome.

Cystic fibrosis is an inherited disease of the exocrine glands and exocrine sweat glands which primarily affects the digestive and respiratory systems. This disease is usually characterised by chronic respiratory infections, pancreatic insufficiency, abnormally viscid mucuous secretions and premature death. The main clinical effects of cystic fibrosis are observed in evidence of respiratory tract involvement and pancreatic insufficiency characterised by failure to grow despite an excellent appetite, frequent foul stools and abnormal pancreatic function tests.

Short bowel syndrome generally refers to a condition in which less than 150 cm of remaining small bowel is associated with a massive loss of absorptive capacity. It is characterised by severe diarrhoea and malabsorption. Patients with short bowel syndrome often experience malabsorption of protein, carbohydrate and fat resulting in calorie depletion and steatorrhea.

The most important therapeutic objective in the management of short bowel is to maintain the patient's nutritional status. By necessity, it is achieved primarily by parenteral nutrition support in the early postoperative period. Enteral nutrition support can be started early after operation when the ileus has resolved. Maximisation of enteral absorption of nutrients is important for long-term survival. Generally, such maximisation requires that the enteral intake greatly exceed the absorptive needs to ensure that the nutritional requirements are met.

The method of the invention can involve the use of a dietary supplement comprising at least 10⁶ metabolically active cells of the genus *Propionibacterium,* at least 0.5 g of a prebiotic that yields a propionate to acetate ratio which is at least 25% higher than that observed for a glucose control in an *in vitro* incubation test as defined appendix I, and a nutritionally acceptable carrier. The dietary supplement may suitably contain at least 0.5 g of a prebiotic as described herein before. Preferably the amount of prebiotic contained in the supplement is at least 1 g, more preferably at 5 g. Usually the amount of prebiotic in a daily unit dosage of the supplement will not exceed 50 g, preferably it will not exceed 30g.

As explained before, the combination of *Propionibacterium* with *Lactobacillus* and/or *Bifidobacterium* strains offers the advantage that it enables very high energy recovery rates. Particularly high energy recovery rates may be achieved by including metabolically active cells of both *Lactobacillus and Bifidobacterium.* Consequently, in a preferred embodiment, the supplement additionally contains at least 10⁶ metabolically active cells of the genus *Bifidobacterium* and at least 10⁶ metabolically active cells of the genus *Lactobacillus.*

In order to achieve good results within a short time frame, it is preferred to incorporate at least 10⁸ metabolically active cells of *Propionibacterium* and at least 10⁸ metabolically active cells of *Lactobacillus and*/*or* at least 10⁸ metabolically active cells of *Bifidobacterium.*

The invention is illustrated by means of the following examples.

### Example 1

A powdered product, packed in a sachet containing 85 g, to be made up with whole milk. For children of 1-5 years of age ½ sachet is made up with 240 ml milk. For children of 5-10 years 1 sachet is made up with 240 ml milk. Servings: 1 per day. For children of 10 years and upward 1-2 servings a day are recommended.

Several flavours may be used, for instance vanilla, strawberry, orange, lemon, mocha or chocolate. (This is also true for most of the other examples)

Per sachet the composition is as follows:
- Energy: 1831 kJ
- Protein: 4g
- Carbohydrate 55g, of which sugars are 20.1 g
- Fat 21 g of which saturated fat 9 g
- Sodium 119 mg
- Potassium 485 mg
- Calcium 111 mg
- Phosphorus 340 mg
- Magnesium 12 mg
- *Propionibacterium freudenreichii spp freudenreichii:* 10⁹ cells
- *Lactobacillus rhamnosus:* 10⁹ cells
- Resistant starch or inulin: 1 g
- Moisture

### Example 2

A powder that can be easily incorporated into an infant milk formula, moist food or a liquid by sprinkling on the recommended amount, and subsequent stirring. It is recommended to paste with a small amount of liquid/water before adding in order to ensure thorough mixing. Dose ranges from 1 g to 10 g/100 ml liquid and should be gradually increased.

The powder is packed in cans in aluminum foil. The composition per 100 g of powder is as follows:
- Energy 2000 kJ
- Protein 0 g
- Carbohydrate 69.1 g; sugars 6.2 g and oligosaccharides 5 g (TOS + inulin ratio 9/1 w/w)
- Fat 21.2 g of which saturated 13.8 g monounsaturated 2.6 g, and polyunsaturated 4.8 g
- Fat contains 35% medium chain triglycerids
- Minerals 20 mg Na, 5 mg K, 20 mg Cl, 5 mg and 5 mg P
- *Propionibacterium acidpropioni:* 10⁹ cells
- *Lactobacillus rhamnosur:* 10⁹ cells
- *Bofidobacterium bifidum* 10⁹ cells

### Example 3

Powder, packed in cans or sachets, used to fortify drinks and foods. Also to be used in tube and sip feeds. Can be mixed with water to form a bland tasting, high energy liquid. Paste the powder with a small amount of water than add the remaining water briskly with a fork. Once prepared, store in refrigerator forty 12 h.

To be introduced into the diet gradually: 1 to 10 gr per 100 ml drink or food
Composition per 100 g:
- Energy 1940 kJ
- Carbohydrate 70 g of which 4.8 g sugar
- Fat: 22 g of which saturated 17.8 g, monounsaturated 0.7 g and polyunsaturated 2.5 g
- Fat is present in the form of 83% MCT and 17% LCT
- 30 mg Na, 3.7 mgK, 50 mg Cl
- *Propionibaeterium freudenreichii spp shermanii:* 10⁸ cells
- *Lactobacillus casei:* 5.10⁹ cells
- Inulin 5 g

### Example 4

Ready to eat, snack sized chunky bar, in the flavour natural or strawberry.
Composition per 100 g
- Energy 2477 kJ
- Protein less than 0.05 g
- Carbohydrate: 47.36 g (sugar)
- Fat 47.36 g of which saturated 28.9 g, monounsaturated 16.3 g, polyunsaturated 2.2 g
- *Propionibacterium freudenreichii spp freudenreichii:* 2.10⁷ cells
- Guar gum: 8 g

### Example 5

Powder in can. Dose 50 g/day for children, 100 g/day for adults. 5 g per spoon.
Sprinkle mix to moist food or liquid. Composition per 100 g:
- Energy 1615 kJ
- Carbohydrates 95 g of which 1.9 g glucose, 4.3 g maltose and 88.8 g polysaccharide including 10 g inulin
- *Propionibacterium acidipropioni:* 10⁹ cells
- *Lactobacillus acidophilus:* 10⁹ cells

### Example 6

Supplement: Powder in a gelatine capsule, tablet or sachet. 0.5 g powder per capsule or tablet Dose: 2 capsules a day Not to be ingested together with hot drinks.
Composition of capsule/tablet:
- *L. rhamnosus* 10¹⁰ cells
- *P. freudenreichii* 10⁸ cells
- *B. bifidum* cells 10⁹ cells
- 0.4 g rhamnose (Sachets contain 1.9 g rhamnose)

### Example 7

Supplement: Capsule, tablet or powder in a sachet. Composition capsule/tablet:
- *Lactobacillus, rhamnosus* 10⁹ cells
- *Propionibacterium, jensenii* 10⁹ cells
- *Lactococcus, lactis ssp cremoris* 10⁹ cells
- *Streptococcus, thermophilus ssp salivarius* 10⁹ cells
- *Bifidobacterium, bididum* 10⁹ cells
- *Lactobacillus fermentum* 10⁹ cells
- 0,5 g guar gum (Sachets contain 1.9 g guar gum)

### APPENDIX I - Protocol for fermentation in dialysis tubes

### Materials

| Micro-organisms | Fresh faeces | |
|---|---|---|
| Substrate | Prebiotics or glucose | |
| McBain & MacFarlane medium | Buffered peptone water | 3.0 g/l |
| | Yeast extract | 2.5 g/l |
| *Fill 500 ml Scott bottles with the medium and* | Mucin (brush borders) | 0.8 g/l |
| *sterilise 15 minutes at 121°C* | Tryptone | 3.0 g/l |
| | L-Cysteine-HCL | 0.4 g/l |
| | Bile salts | 0.05 g/l |
| | K₂HPO₄.3H₂O | 2.6 g/l |
| | NaHCO₃ | 0.2 g/l |
| | NaCl | 4.5 g/l |
| | MgSO₄·7H₂O | 0.5 g/l |
| | CaCl₂ | 0.228 g/l |
| | FeSO₄.7H₂O | 0.005 g/l |
| Buffered medium . | K₂HPO₄.3H₂O | 2.6 g/l |
| | NaHCO₃ | 0.2 g/l |
| *Adjust pH to 6.3 ± 0.1 with* K₂HPO₄ or NaHCO₃. | NaCl | 4.5 g/l |
| *Fill 500 ml Scott bottles with the medium and* | MgSO₄.7H₂O | 0.5 g/l |
| *sterilise 15 minutes at 121°C* | CaCl₂ | 0.228 g/l |
| | FeSO₄.7H₂O | 0.005 g/l |
| Preservative medium | Buffered peptone | 20.0 g/l |
| | L-Cysteine-HCl | 0.5 g/l |
| *Adjust pH to 6.7 ± 0.1 with 1M NaOH or HCl* | Sodium thioglycollate | 0.5 g/l |
| *Boil in microwave.* | Resazurine tablet | 1 per liter |
| *Fill 30 ml serum bottles with 25 ml medium.* | | |
| *Sterilise 15 minutes at 121°C.* | | |

### Methods

### Preservation

The fresh faeces are mixed with the preservative medium and can be preserved in this form for several hours at 4°C.

### Faecal suspension

The preserved solution of faeces is centrifuged at 13,000 rpm for 15 minutes. The supernatant is removed and the faeces are mixed with the McBain and MacFarlane medium in a weight ration of 1:5.

### Fermentation

- Combine 15 ml of the faecal suspension with 500 mg glucose or prebiotic in a bottle and mix thoroughly
- Bring 15 ml of the resulting suspension in a dialysis tube and remove air
- Put the dialysis tube in a 250 ml bottle filled with 250 ml of the buffered medium
- Close the bottles well and incubate at 37°C
- Take a samples from the medium with a hypodermic syringe after 24 and 48 hours (± 1 ml) and immediately put it on ice to stop fermentation

### Determination of the priopionate to acetate ratio

Prepare the samples for gaschromatographic analysis (including methylation of the acids present therein) and determine the relative amounts of propionate and acetate found in each of the prebiotic samples as well as the glucose sample. Calculate the propionate:acetate ratio for each sample and compare the ratios obtained for the prebiotic samples with the ratio observed for the glucose sample.

## Claims

1. Use of a prebiotic that stimulates gastrointestinal activity of *Propionibacterium* in the manufacture of a dietary supplement for use in a method of preventing or alleviating symptoms of malabsorption and/or treating cystic fibrosis in a monogastric mammal, said method comprising the enteral administration to the mammal of an effective amount of a prebiotic selected from the group consisting of inulin; modified inulin; hydrolysed inulin; fructo-oligosaccharides; galactomannan and hydrolysates thereof; arabinogalactan and hydrolysates thereof; trans-galacto-oligosaccharides; rhamnose; pectin and pectin hydrolysates; resistant starch and hydrolysates thereof; indigestible dextrin and hydrolysates thereof; indigestible polydextrose; beta-glucan and hydrolysates thereof; and combinations of these oligo- and polysaccharides.

2. Use according to claim 1 wherein the monogastric mammal is suffering from impaired intestinal digestion which is causing malabsorption.

3. Use according to claim 1 or 2, wherein the malabsorption is associated with a disorder selected from the group consisting of cystic fibrosis, pancreatic insufficiency, pancreatitis and short bowel syndrome.

4. Use according to any one of claims 1-3, wherein the malabsorption is caused by impaired intestinal digestion of protein, carbohydrates and/or fat.

5. Use according to any one of claims 1-4, wherein the method comprises enteral administration of a combination of one or more strains of the genus *Propionibacterium.*

6. Use according to claim 5, wherein the method comprises the enteral administration of one or more metabolically active strains of the genus *Propionibacterium* in a daily amount of at least 10⁶ cells.

7. Use according to claim 5 or 6, wherein the one or more strains of the genus *Propionibacterium* are selected from the group consisting of *P. freudenreichii, spp freudenreichii* or *spp shermanii; P. jensenii; P. acidipropionici* and *P. microaerophilus.*

8. Use according to any one of claims 1-7, wherein the prebiotic is administered in a daily amount of between 0.5 and 50 g.

9. Use according to any one of claims 1-8, wherein the prebiotic yields a propionate to acetate ratio which is at least 25% higher than that observed for a glucose control in an *in vitro* incubation test as defined in the description.

10. Use according to any one of claims 1-9, wherein the prebiotic comprises resistant starch.

11. Use according to any one of claims 1-10, wherein the prebiotic comprises inulin.

12. Use according to any one of claims 1-11, wherein the method comprises the enteral co-administration of one or more metabolically active homofermentative strains of the genus *Lactobacillus,* preferably in a daily amount of at least 10⁶ cells.

13. Use according to any one of claims 1-12, wherein the method comprises the enteral co-administration of one or more metabolically active strains of the genus *Bifidobacterium,* preferably in a daily amount of at least 10⁶ cells.

## Patentansprüche

1. Verwendung eines Präbiotikums, das die gastrointestinale Aktivität vom *Propionibakterium* unterstützt, zur Herstellung eines Nahrungsergänzungsmittels zur Verwendung in einem Verfahren zur Vorbeugung oder Abschwächung von Symptomen der Malabsorption und/oder Behandlung zystischer Fibrose in einem monogastrischen Säugetier, wobei das Verfahren die enterale Verabreichung einer wirksamen Menge eines Präbiotikums, ausgewählt aus der Gruppe bestehend aus Inulin, modifiziertem Inulin, hydrolisiertem Inulin, Frukto-Oligosaccharide, Galaktomannan und Hydrolysaten davon, Arabinogalaktan und Hydrolysaten davon, Trans-Galakto-Oligosacchariden, Rhamnose, Pektin und Pektin-Hydrolysaten, resistenter Stärke und Hydrolysaten davon, unverdaubarem Dextrin und Hydrolysaten davon, unverdaubarer Polydextrose, Beta-Glucan und Hydrolysaten davon und Kombinationen dieser Oligo- und Polysaccharide, an das Säugetier umfasst.

2. Verwendung nach Anspruch 1, wobei das monogastrische Säugetier an einer eingeschränkten intestinalen Verdauung leidet, die Malabsorption verursacht.

3. Verwendung nach Anspruch 1 oder 2, wobei die Malabsorption mit einer Krankheit assoziiert ist, ausgewählt aus der Gruppe bestehend aus zystischer Fibrose, Pankreas-Insuffizienz, Pankreatitis und Kurzdarmsyndrom.

4. Verwendung nach einem der Ansprüche 1 bis 3, wobei die Malabsorption durch eine eingeschränkte intestinale Verdauung von Proteinen, Kohlenhydraten und/oder Fett verursacht wird.

5. Verwendung nach einem der Ansprüche 1 bis 4, wobei das Verfahren die enterale Verabreichung einer Kombination eines oder mehrerer Stämme des Genus *Propionibakterium* umfasst.

6. Verwendung nach Anspruch 5, wobei das Verfahren die enterale Verabreichung eines oder mehrerer metabolisch aktiver Stämme des Genus *Propionibakterium* in einer täglichen Menge von wenigstens 10⁶ Zellen umfasst.

7. Verwendung nach Anspruch 5 oder 6, wobei der eine oder die mehreren Stämme des Genus *Propionibakterium* ausgewählt sind aus der Gruppe bestehend aus: *P. freudenreichii, spp freudenreichii* oder *spp shermanii; P. jensenii; P. acidipropionici* und *P. microaerophilus.*

8. Verwendung nach einem der Ansprüche 1 bis 7, wobei das Präbiotikum in einer täglichen Menge zwischen 0,5 und 50 g verabreicht wird.

9. Verwendung nach einem der Ansprüche 1 bis 8, wobei das Präbiotikum ein Propionat-zu-Acetat-Verhältnis erzielt, das wenigstens 25 % höher ist als das einer Glukosekontrolle in einem *in vitro* Inkubationstest, wie in der Beschreibung definiert.

10. Verwendung nach einem der Ansprüche 1 bis 9, wobei das Präbiotikum resistente Stärke umfasst.

11. Verwendung nach einem der Ansprüche 1 bis 10, wobei das Präbiotikum Inulin umfasst.

12. Verwendung nach einem der Ansprüche 1 bis 11, wobei das Verfahren die enterale CoVerabreichung eines oder mehrerer metabolisch aktiver homofermentativer Stämme des Genus *Lactobacillus* umfasst, vorzugsweise in einer täglichen Menge von wenigstens 10⁶ Zellen.

13. Verwendung nach einem der Ansprüche 1 bis 12, wobei das Verfahren die enterale CoVerabreichung eines oder mehrerer metabolisch aktiver Stämme des Genus *Bifidobacterium* umfasst, vorzugsweise in einer täglichen Menge von wenigstens 10⁶ Zellen.

## Revendications

1. Utilisation d'un prébiotique qui stimule l'activité gastro-intestinale de *Propionibacterium* dans la production d'un complément alimentaire destiné à être utilisé dans une méthode pour prévenir ou soulager les symptômes de malabsorption et/ou traiter la fibrose kystique chez un mammifère monogastrique, ladite méthode comprenant l'administration entérale au mammifère d'une quantité efficace d'un prébiotique choisi dans le groupe consistant en l'inuline ; l'inuline modifiée ; l'inuline hydrolysée ; des fructo-oligosaccharides ; le galactomannane et ses hydrolysats ; l'arabinogalactane et ses hydrolysats ; des transgalacto-oligosaccharides ; le rhamnose ; la pectine et des hydrolysats de pectine ; l'amidon résistant et ses hydrolysats ; la dextrine non digestible et ses hydrolysats ; le polydextrose non digestible ; le bêta-glucane et ses hydrolysats ; et des associations de ces oligo- et polysaccharides.

2. Utilisation suivant la revendication 1, dans laquelle le mammifère monogastrique souffre d'une digestion intestinale entravée qui provoque une malabsorption.

3. Utilisation suivant la revendication 1 ou 2, dans laquelle la malabsorption est associée à un trouble choisi dans le groupe consistant en la fibrose kystique, l'insuffisance pancréatique, la pancréatite et le syndrome de restriction intestinale étendue.

4. Utilisation suivant l'une quelconque des revendications 1 à 3, dans laquelle la malabsorption est provoquée par une digestion intestinale entravée de protéines, de glucides et/ou de matières grasses.

5. Utilisation suivant l'une quelconque des revendications 1 à 4, dans laquelle la méthode comprend l'administration entérale d'une association d'une ou plusieurs souches du genre *Propionibacterium.*

6. Utilisation suivant la revendication 5, dans laquelle la méthode comprend l'administration entérale d'une ou plusieurs souches métaboliquement actives du genre *Propionibacterium* en une quantité quotidienne d'au moins 10⁶ cellules.

7. Utilisation suivant la revendication 5 ou 6, dans laquelle la ou les souches du genre *Propionibacterium* sont choisies dans le groupe consistant en *P*. *freudenreichii, ssp. freudenreichii* ou *spp. shermanii ; P. jensenii ; P. acidipropionici* et *P*. *microaerophilus.*

8. Utilisation suivant l'une quelconque des revendications 1 à 7, dans laquelle le prébiotique est administré en une quantité quotidienne de 0,5 à 50 g.

9. Utilisation suivant l'une quelconque des revendications 1 à 8, dans laquelle le prébiotique donne un rapport du propionate à l'acétate qui est supérieur d'au moins 25 % à celui observé pour un témoin au glucose dans un test d'incubation *in vitro* tel que défini dans la description.

10. Utilisation suivant l'une quelconque des revendications 1 à 9, dans laquelle le prébiotique comprend de l'amidon résistant.

11. Utilisation suivant l'une quelconque des revendications 1 à 10, dans laquelle le prébiotique comprend de l'inuline.

12. Utilisation suivant l'une quelconque des revendications 1 à 11, dans laquelle la méthode comprend la coadministration entérale d'une ou plusieurs souches homofermentatives métaboliquement actives du genre *Lactobacillus,* de préférence en une quantité quotidienne d'au moins 10⁶ cellules.

13. Utilisation suivant l'une quelconque des revendications 1 à 12, dans laquelle la méthode comprend la coadministration entérale d'une ou plusieurs souches métaboliquement actives du genre *Bifidobacterium,* de préférence en une quantité quotidienne d'au moins 10⁶ cellules.
